(19) Europäisches Patentamt
European Patent Office
Office européen des brevets

(11) Veröffentlichungsnummer: **0 441 187 A2**

# (12) EUROPÄISCHE PATENTANMELDUNG

(21) Anmeldenummer: **91100878.7**

(51) Int. Cl.⁵: **A61K 6/00**

(22) Anmeldetag: **24.01.91**

(30) Priorität: **06.02.90 DE 4003435**

(43) Veröffentlichungstag der Anmeldung:
**14.08.91 Patentblatt 91/33**

(84) Benannte Vertragsstaaten:
**AT BE CH DE DK ES FR GB GR IT LI LU NL SE**

(71) Anmelder: **BAYER AG**

**W-5090 Leverkusen 1 Bayerwerk(DE)**

(72) Erfinder: **Müller, Michael, Dr.**
**Richard-Zanders-Strasse 34**
**W-5060 Bergisch Gladbach 2(DE)**
Erfinder: **Podszun, Wolfgang, Dr.**
**Roggendorfstrasse 55**
**W-5000 Köln(DE)**

(54) **Carbonsäure-(meth)acryloylaminoalkylester in Adhäsivkomponenten zur Behandlung kollagenhaltiger Materialien.**

(57) Die Erfindung betrifft Zubereitungen zur Verwendung als Adhäsivkomponente zur Behandlung kollagenhaltiger Materialien, Verfahren zur Herstellung und die Verwendung dieser Zubereitungen.

EP 0 441 187 A2

# CARBONSÄURE-(METH)ACRYLOYLAMINOALKYLESTER IN ADHÄSIVKOMPONENTEN ZUR BEHANDLUNG KOLLAGENHALTIGER MATERIALIEN

Die Erfindung betrifft Zubereitungen zur Verwendung als Adhäsivkomponente zur Behandlung kollagenhaltiger Materialien, Verfahren zur Herstellung und die Verwendung dieser Zubereitungen.

Die beanspruchten Zubereitungen enthalten Carbonsäure-(meth)acryloylaminoalkylester (I) der Formel

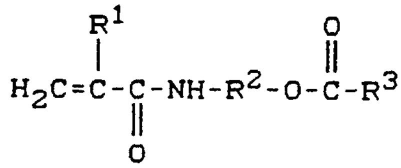

(I),

in der

$R^1$

Wasserstoff oder Methyl bedeutet,

$R^2$

Methylen oder Ethylen bedeutet

und

$R^3$

für Wasserstoff, Methyl oder Ethyl steht und gegebenenfalls Zusätze wie Initiatoren, Lösungsmittel und Füllstoffe.

Kollagenhaltige Materialien sind Gerüsteiweißkörper und Hauptbestandteile dar menschlichen und tierischen interzellularen Stützsubstanzen wie Knorpel- und Knochengewebe, Haut und Zahnbein (Dentin). Im Rahmen der vorliegenden Erfindung werden die Adhäsivkomponenten bevorzugt zur Behandlung von Dentin im Zusammenhang mit Zahnreparaturen verwandet.

Besonders im Dentalbereich werden härtende, polymere Materialien als Füllmaterialien bei Zahnreparaturen verwendet. Als härtende, polymere Materialien werden im allgemeinen Füllungen auf Acrylatbasis bevorzugt. Diese polymeren Füllungen haben jedoch den Nachteil, daß sie schlecht am Dentin haften bleiben. Um dieses Problem zu lösen, hat man bisher teilweise Unterschneidungen am Zahnbein vorgenommen; dazu war es erforderlich, über den angegriffenen Bereich hinaus, beachtliche Mengen an gesundem Dentin zu entfernen.

Nach einer anderen Methode ätzt man das Dentin und die Schmelzoberfläche mit Säuren, wie z.B. Phosphorsäure, an, und nimmt dann die Füllung vor. Abgesehen davon, daß die Säure eine Reizwirkung im Mundbereich ausübt, dringt sie auch leicht durch die Dentinkanäle in den Zahn und schädigt den Nerv (Pulpa).

In J. Dent. Res. 57, 500-505 (1978), werden aldehydgruppenhaltige Methacrylate der isomeren Hydroxybenzaldehyde beschrieben, die als Grundierungsmittel für Füllungen im Dentalbereich verwendet werden können. Die Bindung zwischen Dentin und Füllmasse bleibt jedoch auch nach einer solchen Grundierung unbefriedigend.

In Scand. J. Dent. Res. 92, 980-983 (1984) und J. Dent. Res. 63, 1087-1089 (1984) werden Grundierungsmittel aus wäßrigem Formaldehyd oder Glutaraldehyd und $\beta$-Hydroxyethylmethacrylat(HEMA) beschrieben.

Außerdem sind in der EP-A 0 141 324 Zusammensetzungen aus einem Aldehyd und einem olefinisch ungesättigten Monomer mit aktivem Wasserstoff beschrieben, die eine Anbindung an Dentin aufweisen.

Die neuen Zubereitungen auf Basis von Carbonsäure-(meth)acryloylaminoalkylestern (I) bewirken eine starke Verbundhaftung von Materialien, die am Kollagen befestigt werden sollen, z.B. eine Verbundhaftung von Zahnfüllungsmaterial in einer Kavität am Zahn.

Die Carbonsäure-(meth)acryloylaminoalkylester (I) sind bekannte Synthesezwischenstufen und wurden z.B. als Monomerbestandteil zur Herstellung von Copolymeren verwendet (DE-OS 2 217 746, DE-OS 1 927 642, DE-OS 3 619 914).

Carbonsäure-(meth)acryloylaminomethylester wurden durch Veresterung des N-Hydroxymethyl(meth)-acrylamids erhalten (DE-OS 1 927 642, DE-OS 1 281 438). Carbonsäure-(meth)acryloylaminomethylester lassen sich entsprechend aus dem N-2-Hydroxyethyl(meth)acrylamid (JA 60 262 805) herstellen.

Die gute Wirksamkeit der Carbonsäure-(meth)acryloylaminoalkylester (I) in den erfindungsgemäßen

Zubereitungen als Adhäsivkomponente für kollagenhaltige Materialien war überraschend, weil sie keine reaktiven Gruppen enthalten, die unter milden Bedingungen geeignete chemische Bindungen zu kollagenhaltigen Materialien aufbauen können, was bislang als eine wichtige Voraussetzung angenommen wurde (J.C. Sectos, Am. J. Dent. 1. (1988) 173).

Beispielsweise seien die folgenden Carbonsäure-(meth)acryloylaminoalkylester als Bestandteile der erfindungsgemäßen Zubereitungen genannt:

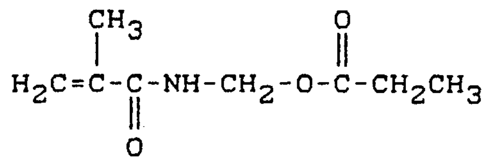

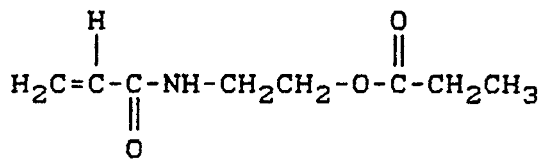

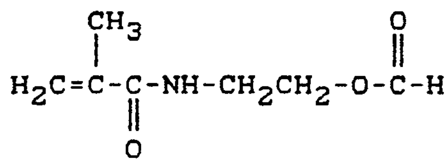

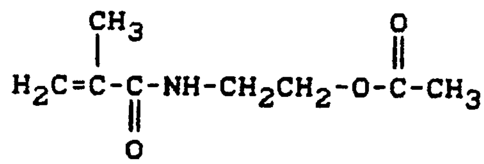

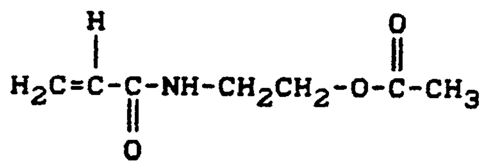

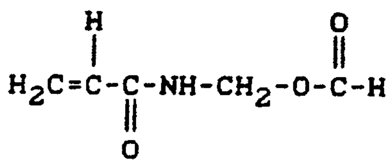

Besonders bevorzugt sind die Carbonsäure-(meth)acryloylaminomethylester der Formeln:

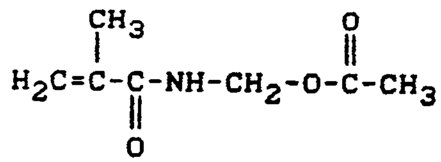

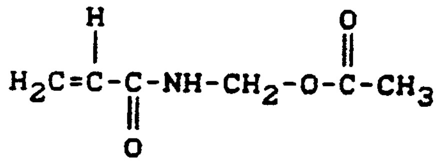

und

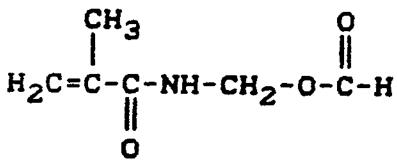

Initiatoren im Rahmen der vorliegenden Erfindung sind Radikalbildner, die eine radikalische Polymerisation auslösen. Bevorzugt sind Fotoinitiatoren, die unter der Einwirkung von Licht, beispielsweise UV-Licht, sichtbarem Licht oder Laserlicht, eine radikalische Polymerisation auslösen.

Die sogenannten Fotopolymerisationsinitatoren sind an sich bekannt (Houben-Weyl, Methoden der organischen Chemie, Band E 20, Seite 80 ff, Georg Thieme Verlag Stuttgart 1987). Vorzugsweise handelt es sich um Mono- oder Dicarbonylverbindungen, wie Benzoin und dessen Derivate, insbesondere Benzoinmethylether, Benzil und Benzilderivate, beispielsweise 4,4-Oxidibenzil und andere Dicarbonylverbindungen wie Diacetyl, 2,3-Pentandion und $\alpha$-Diketo-Derivate des Norbornans und substituierter Norbornane, Metallcarbonyle wie Pentacarbonylmangan oder Chinone wie 9,10-Phenanthrenchinon und Naphthochinon. Besonders bevorzugt ist Campherchinon.

Die erfindungsgemäßen Zubereitungen enthalten im allgemeinen 0,01 bis 2 Gew.-Teile, bevorzugt 0,1 bis 0,5 Gew.-Teile des Initiators, bezogen auf 100 Gew.-Teil an polymerisierbaren Verbindungen. Enthält eines der mit der erfindungsgemäßen Adhäsivkomponente im Kontakt stehenden Fügeteile bereits einen Initiator der beschriebenen Art, kann auf den Initiator in der Adhäsivkomponente auch ganz verzichtet werden.

Die Lösungsmittel im Rahmen der vorliegenden Erfindung sollen die Komponente lösen und sollen, durch die Anwendung bedingt, untoxisch sein. Bevorzugt seien Wasser und flüchtige organische Lösungsmittel wie Methanol, Ethanol, Propanol, Isopropanol, Aceton, Methylethylketon, Essigsäuremethyl- oder -ethylester und Tetrahydrofuran genannt.

Im allgemeinen setzt man 10 bis 1000 Gew.-%, bevorzugt 50 bis 300 Gew.-% des Lösungsmittels, bezogen auf den Carbonsäure-(meth)acryloylaminoalkylester ein.

Es kann vorteilhaft sein, den erfindungsgemäßen Zubereitungen Coaktivatoren zuzusetzen, die die Polymerisationsreaktion beschleunigen. Bekannte Beschleuniger sind beispielsweise Amine wie p-Toluidin, Dimethyl-p-toluidin, Trialkylamine wie Trihexylamin, Polyamine wie N,N,N',N'-Tetraalkylalkylendiamin, Barbitursäure und Dialkylbarbitursäure.

Die Coaktivatoren werden im allgemeinen in einer Menge von 0,02 bis 4 Gew.-%, bevorzugt 0,2 bis 1 Gew.-%, bezogen auf die Menge an polymerisierbaren Verbindungen eingesetzt.

Als weitere Komponente können die erfindungsgemäßen Zusammensetzungen Carbonylverbindungen enthalten.

Carbonylverbindungen im Rahmen der vorliegenden Erfindung sind Aldehyde und Ketone, die 1 bis 20, bevorzugt 1 bis 10, und besonders bevorzugt 2 bis 6 Kohlenstoffatome, enthalten. Die Carbonylfunktion kann an einem aliphatischen, aromatischen oder heterocyclischen Molekülteil gebunden sein.

Als Aldehyde seien aliphatische Mono- oder Dialdehyde genannt. Bevorzugt wird Formaldehyd, Acetaldehyd, Propionaldehyd, 2-Methylpropionaldehyd, Butyraldehyd, Benzaldehyd, Vanillin, Furfural, Anisaldehyd, Salicylaldehyd, Glyoxal, Glutardialdehyd und Phthaldialdehyd. Besonders bevorzugt ist der Glutardialdehyd.

Als Ketone seien besonders aliphatische Mono- und Diketone genannt. Bevorzugt sind Butanon, Aceton,

Cyclooctanon, Cycloheptanon, Cyclohexanon, Cyclopentanon, Acetophenon, Benzophenon, 1-Phenyl-2-propanon, 1,3-Diphenyl-2-propanon, Acetylaceton, 1,2-Cyclohexandion, 1,2-Cyclopentandion und Campherchinon. Besonders bevorzugt ist Cyclopentanon.

Im allgemeinen setzt man 1 bis 1000 Gew.-%, bevorzugt 5 bis 50 Gew.-% der Carbonylverbindungen, bezogen auf den Carbonsäure-(meth)acryloylaminoalkylester ein.

Als weitere Komponente können die erfindungsgemäßen Zusammensetzungen (Meth)-acrylsäureester enthalten, die Vernetzungen ausbilden können. (Meth)-acrylsäureester, die Vernetzungen ausbilden können, enthalten im allgemeinen 2 oder mehr polymerisierbare aktive Gruppen im Molekül. Bevorzugt seien Ester der (Meth)-acrylsäure mit 2- bis 5-wertigen Alkoholen mit 2 bis 30 Kohlenstoffatomen genannt. Besonders bevorzugt werden Alkoxy(meth)acrylate und Urethangruppen enthaltende (Meth)acrylate.

Beispielsweise seien (Meth)acrylsäureester der Formel

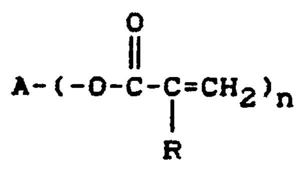

in der

A einen geradkettigen, verzweigten, cyclischen, aliphatischen, aromatischen oder gemischt aliphatischaromatischen Rest mit 2 bis 25 C-Atomen, der durch -O-, NH- oder O-CO-NH-Brücken unterbrochen und mit Hydroxy, Oxy, Carboxy, Amino oder Halogen substituiert sein kann, bedeutet,

R H oder Methyl bedeutet und

n für eine ganze Zahl von 2 bis 8, vorzugsweise 2 bis 4, steht,

genannt.

Vorzugsweise seien Verbindungen der folgenden Formeln genannt:

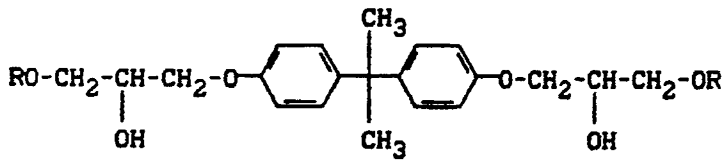

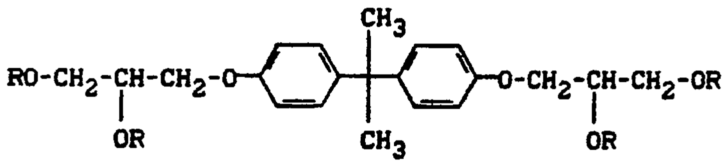

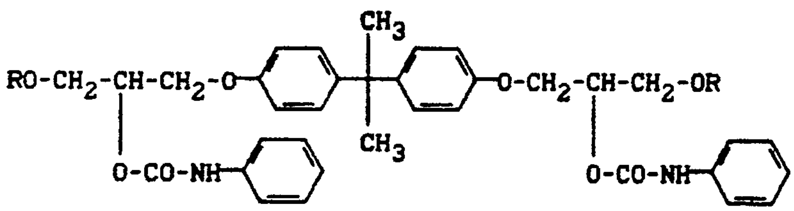

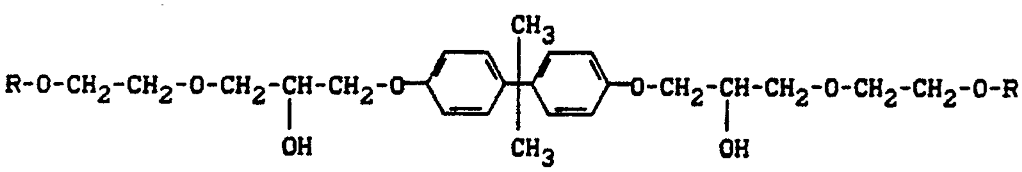

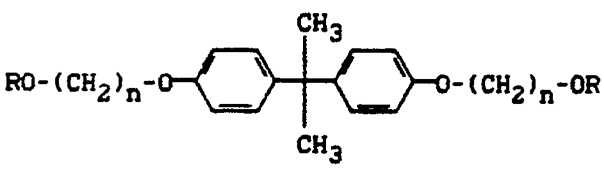

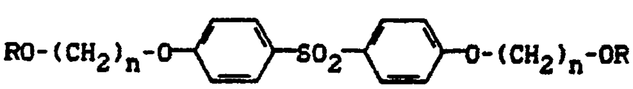

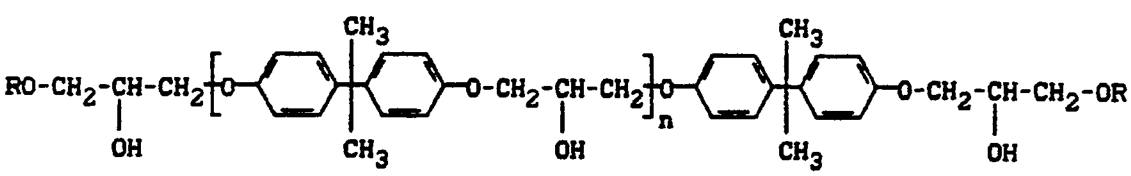

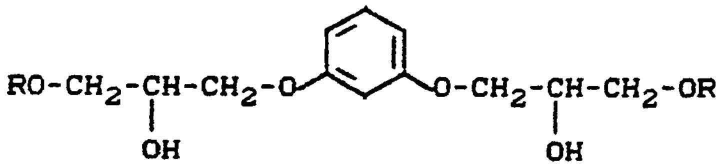

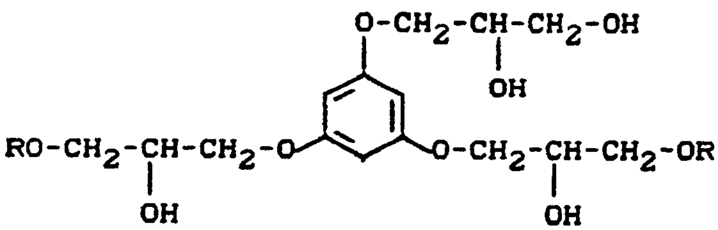

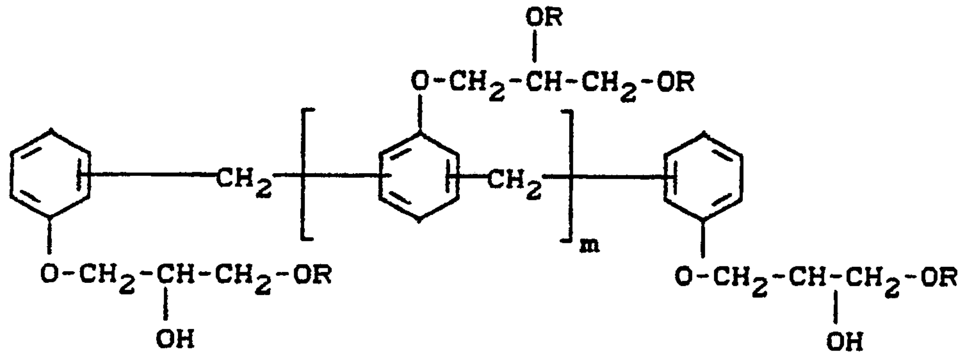

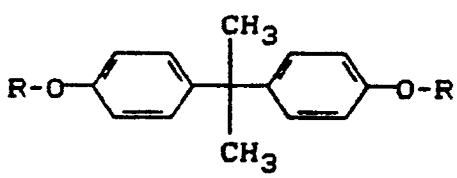

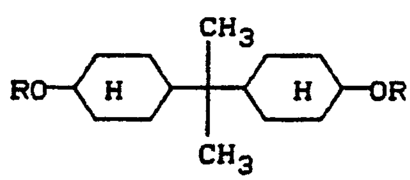

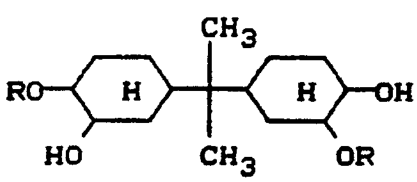

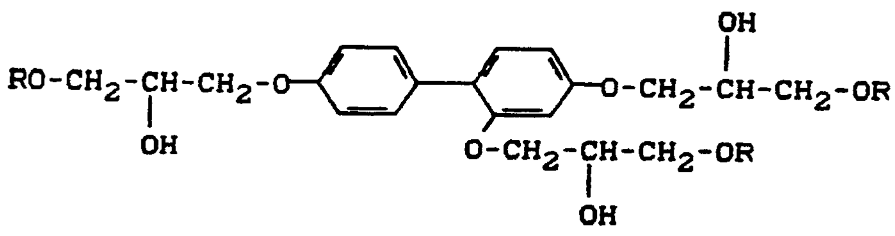

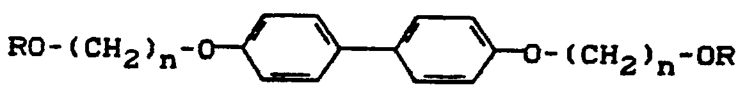

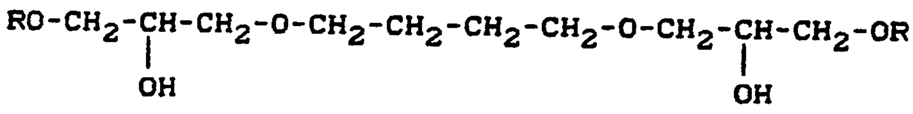

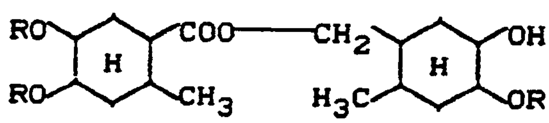

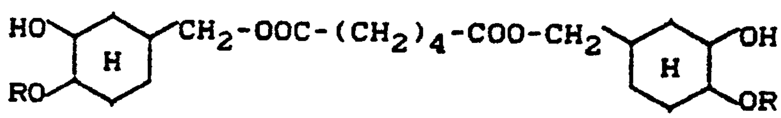

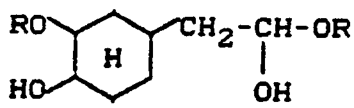

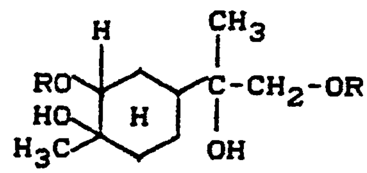

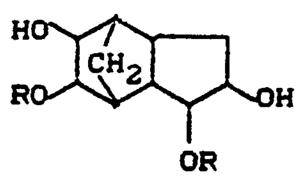

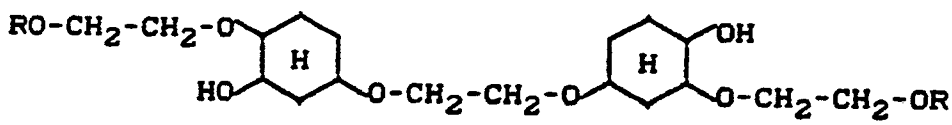

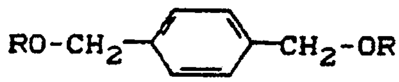

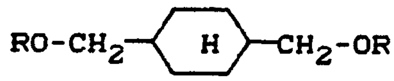

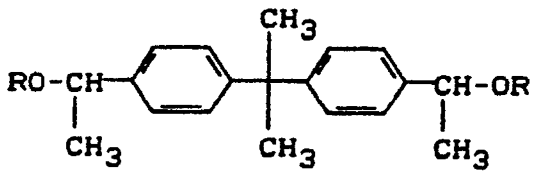

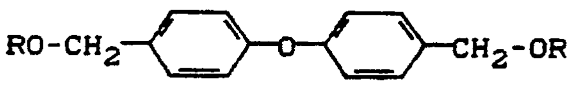

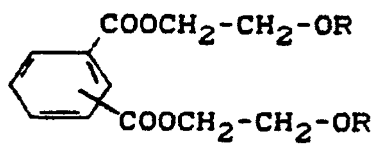

in der ortho-, meta- oder para-Form

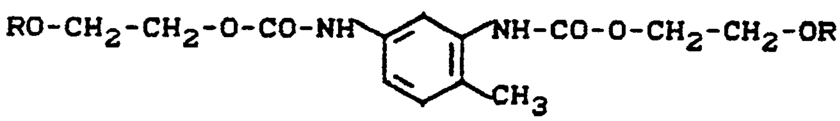

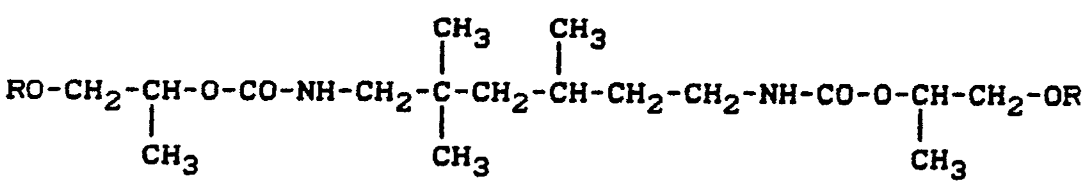

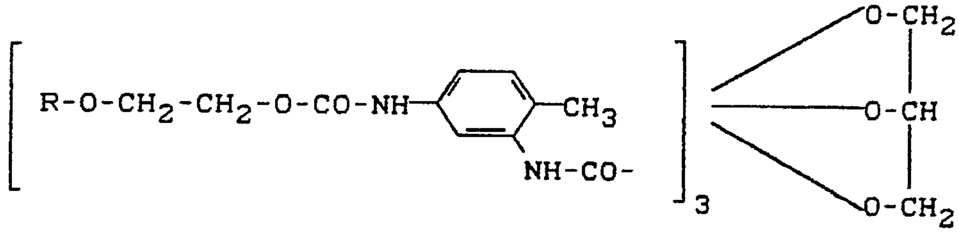

$R^4-O-(CH_2)_n-O-R^4$

$R^4-O-(CH_2CH_2O)_m-R^4$

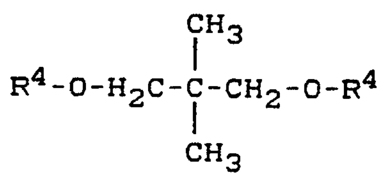

worin

$R^4$ für 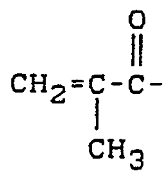 oder 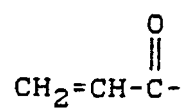 steht,

n eine Zahl von 1 bis 4 und
m eine Zahl von 0 bis 5 bedeutet.

Außerdem seien Derivate des Tricyclodecans (EP-A-00 23 686) und Umsetzungsprodukte aus Polyolen, Diisocyanaten und Hydroxyalkylmethacrylaten (DE-A-37 03 120, DE-A-37 03 080 und DE-A-37 03 130) genannt. Beispielsweise seien die folgenden Monomeren genannt:

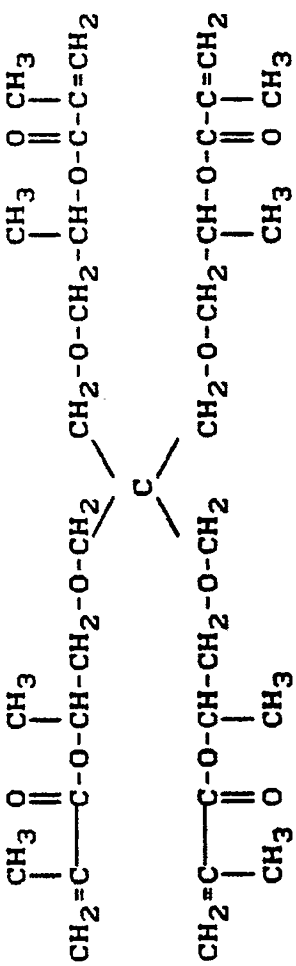

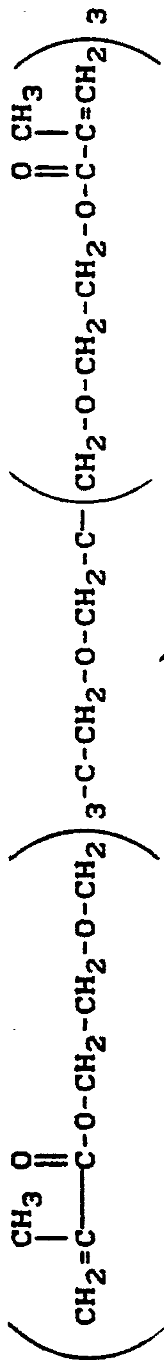

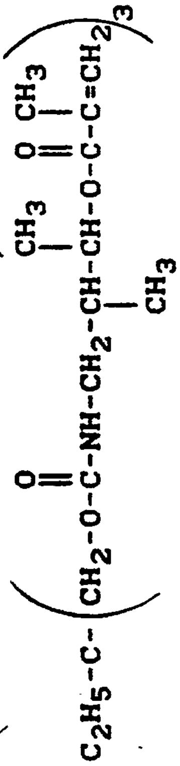

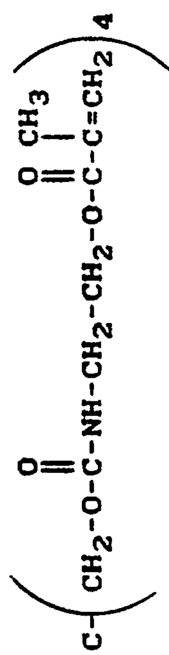

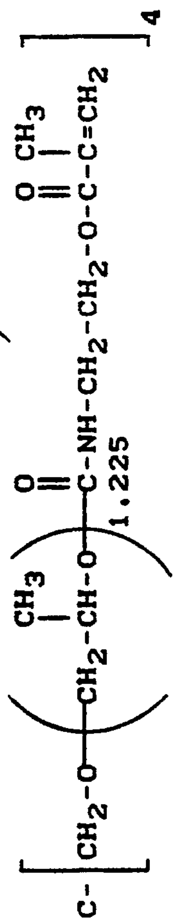

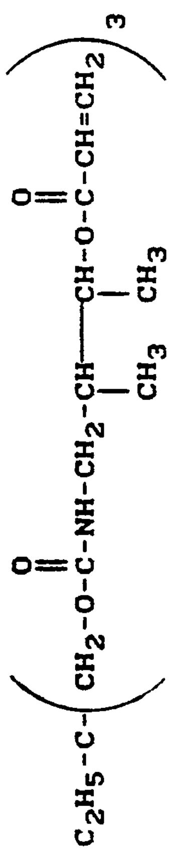

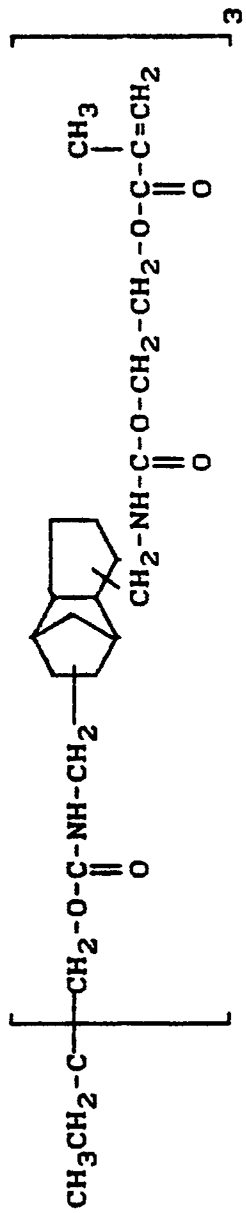

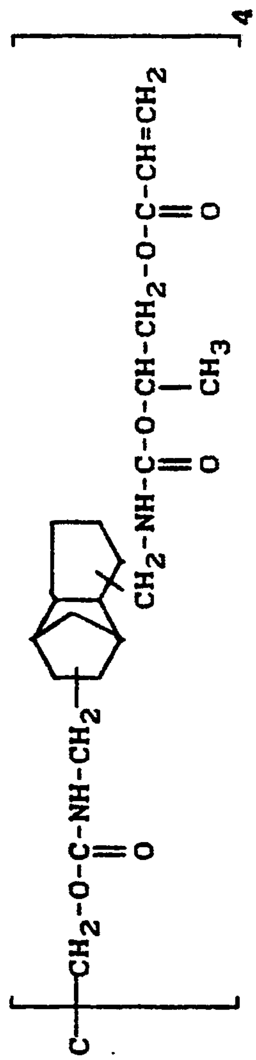

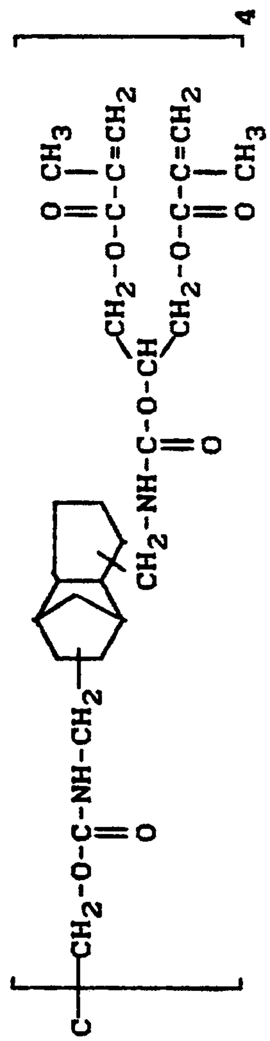

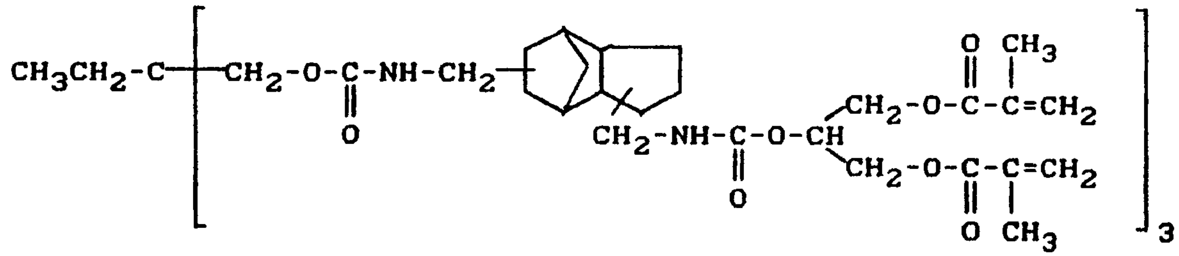

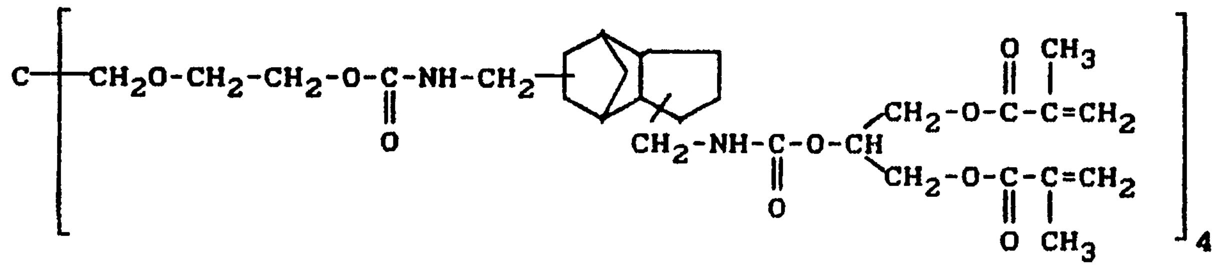

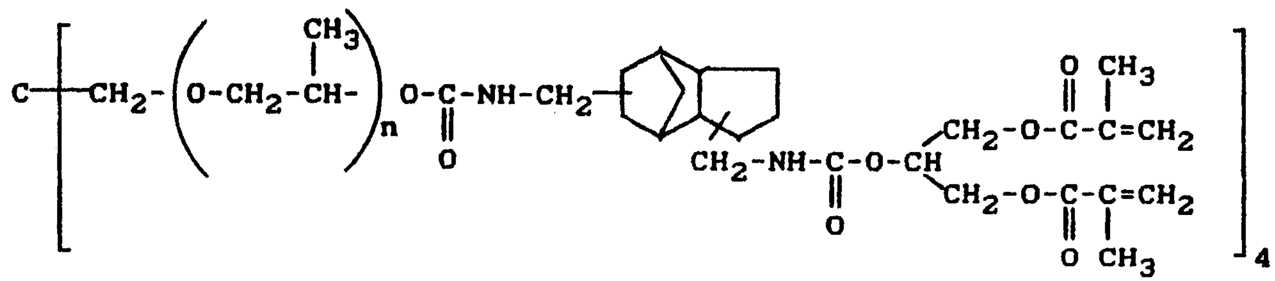

n = 1,225 (statistischer Mittelwert für 4 Ketten)

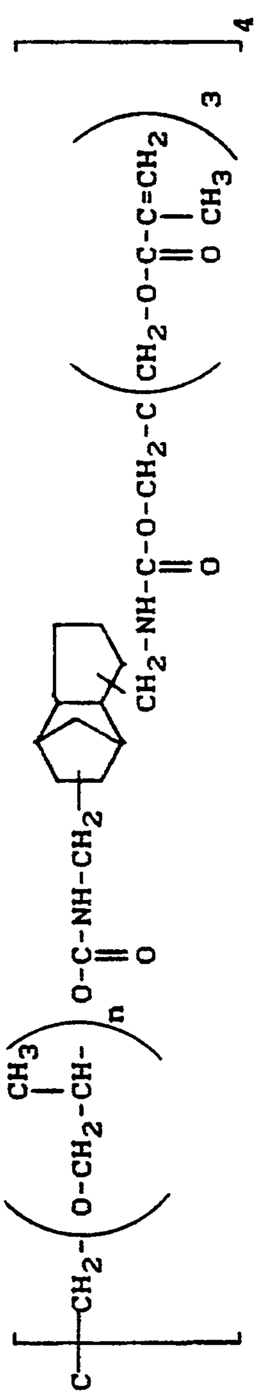

n = 1,225 (Mittelwert)

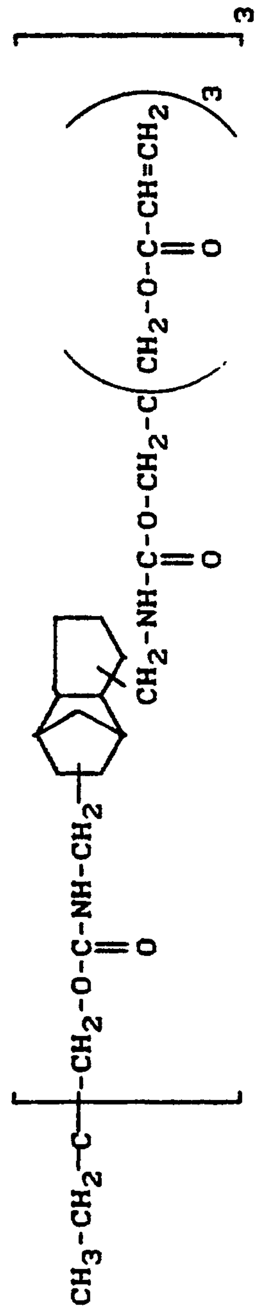

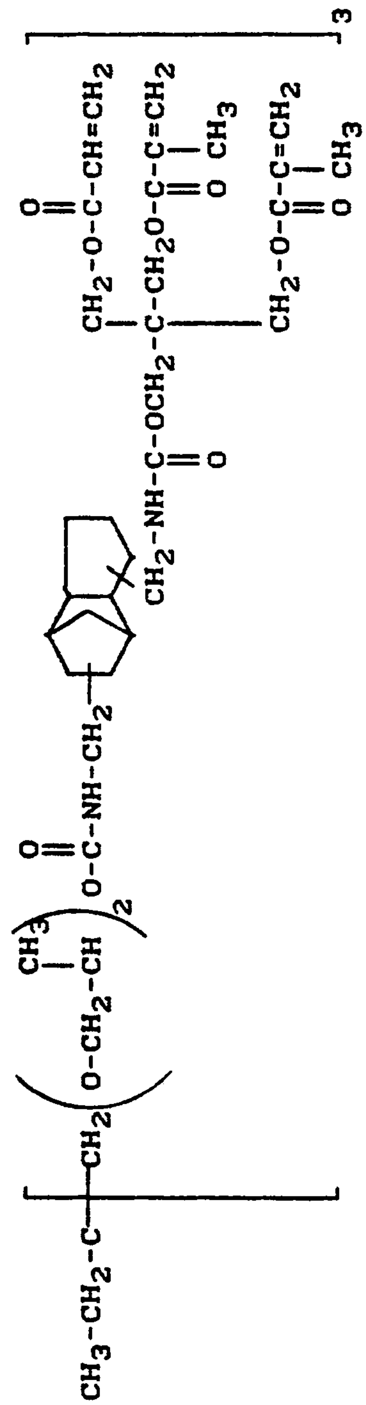

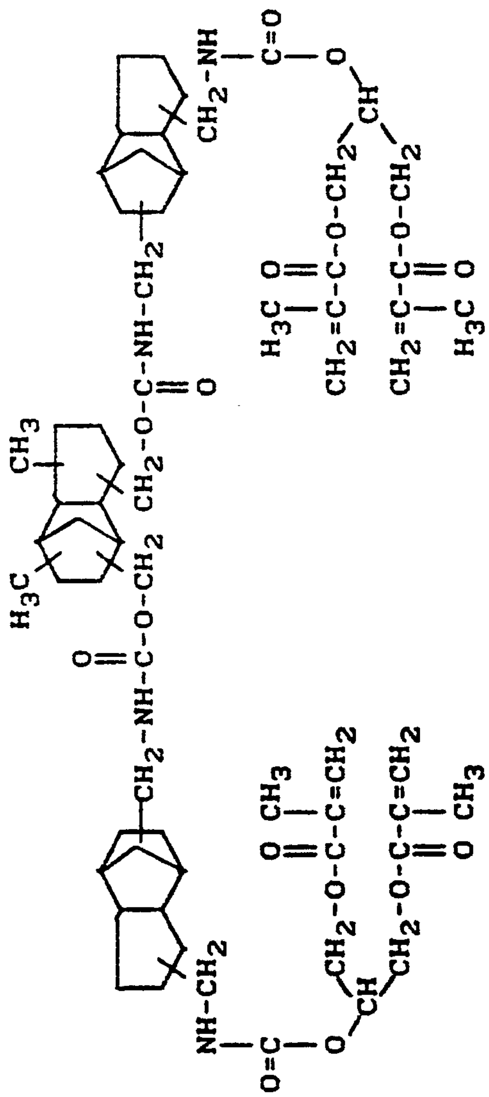

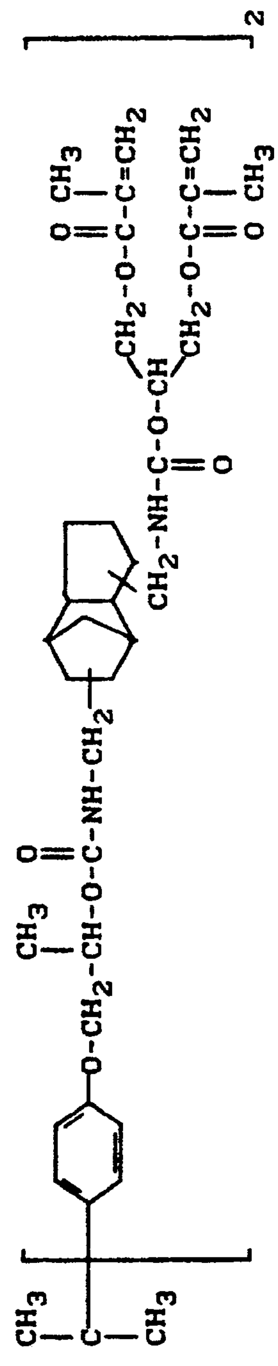

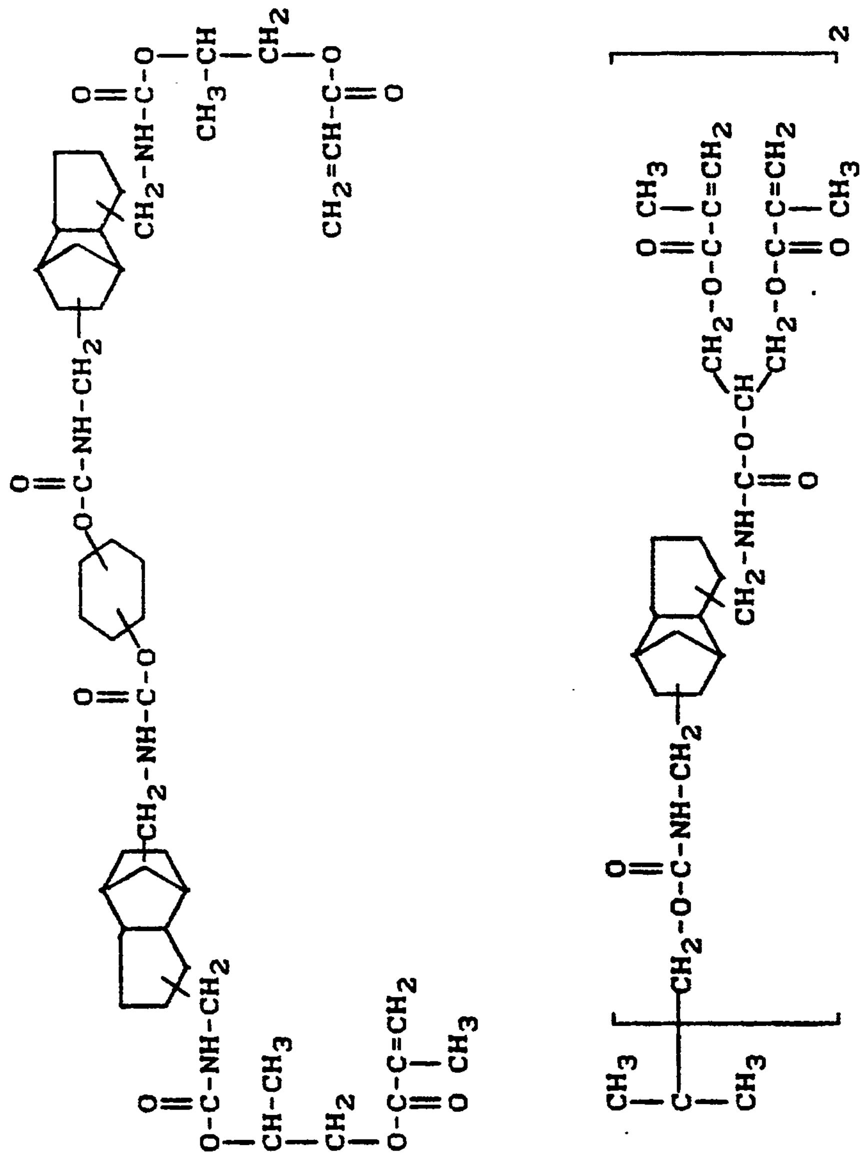

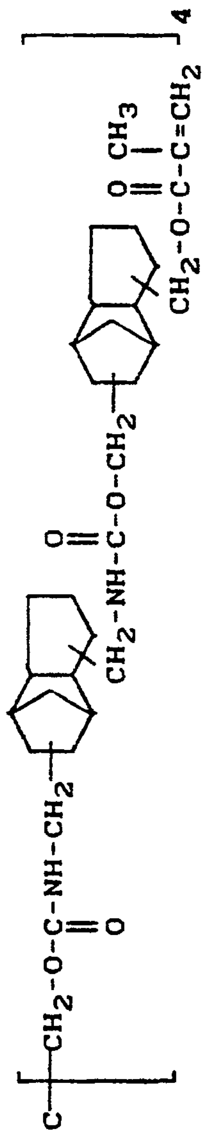

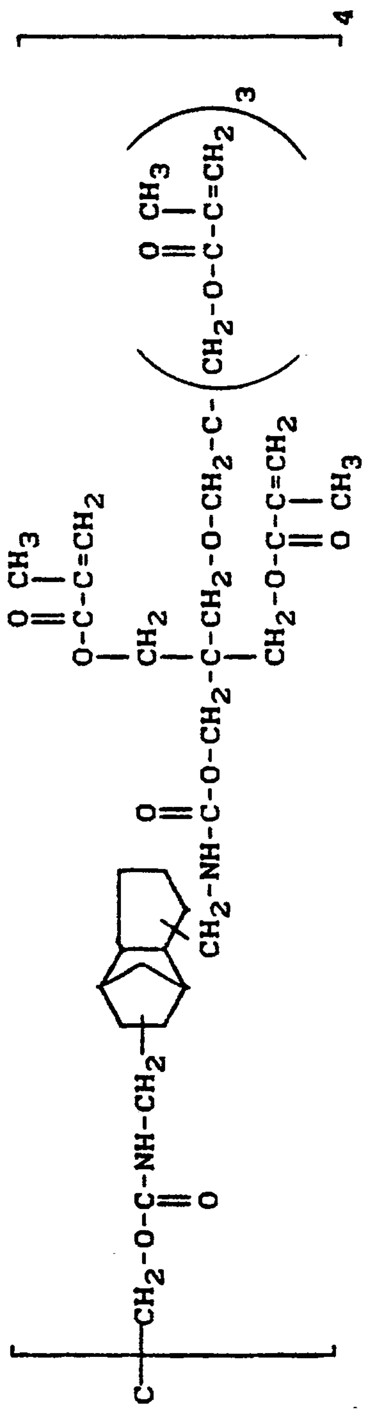

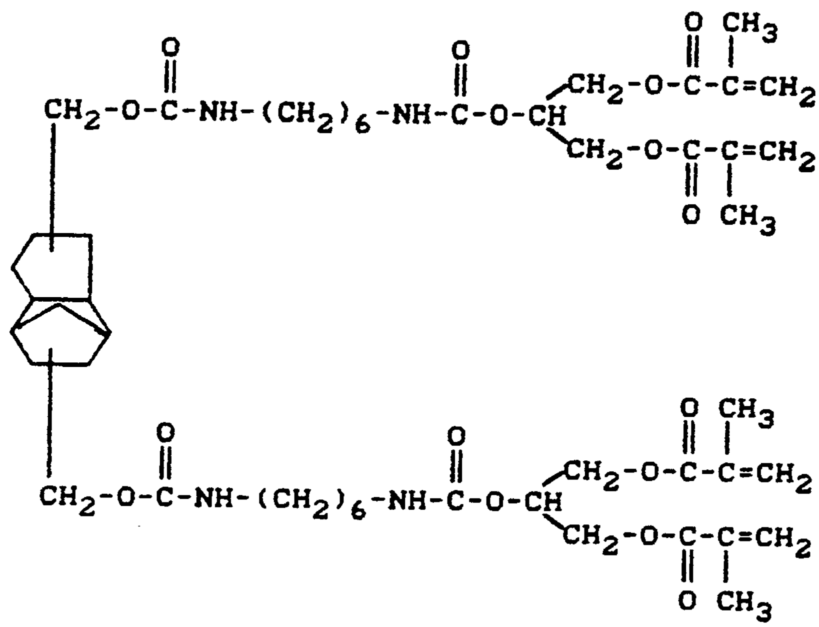

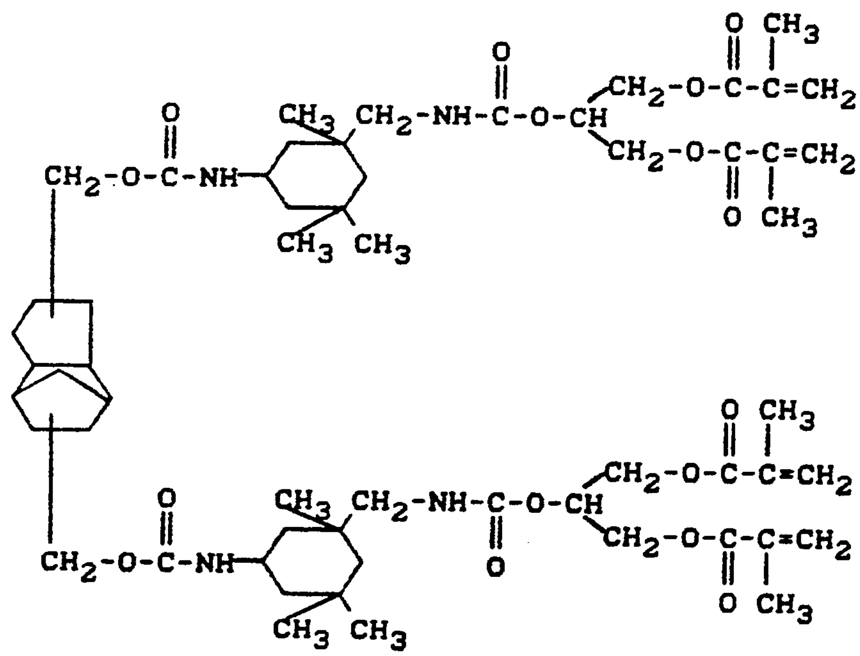

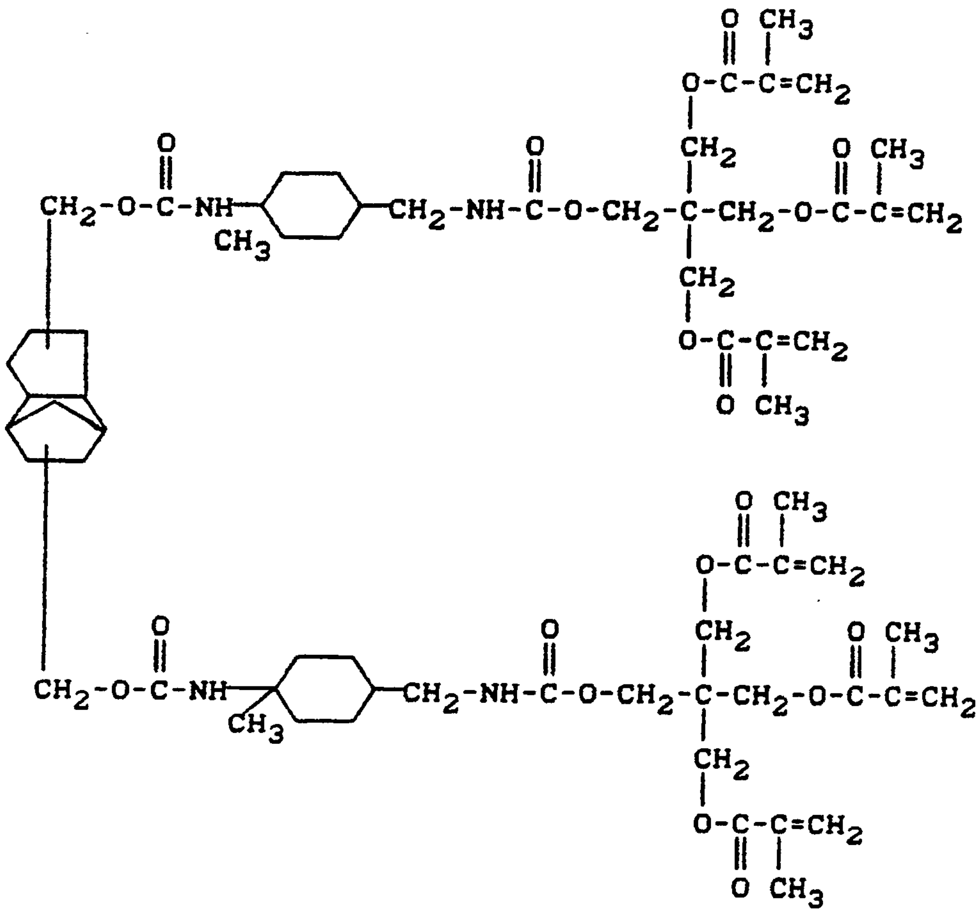

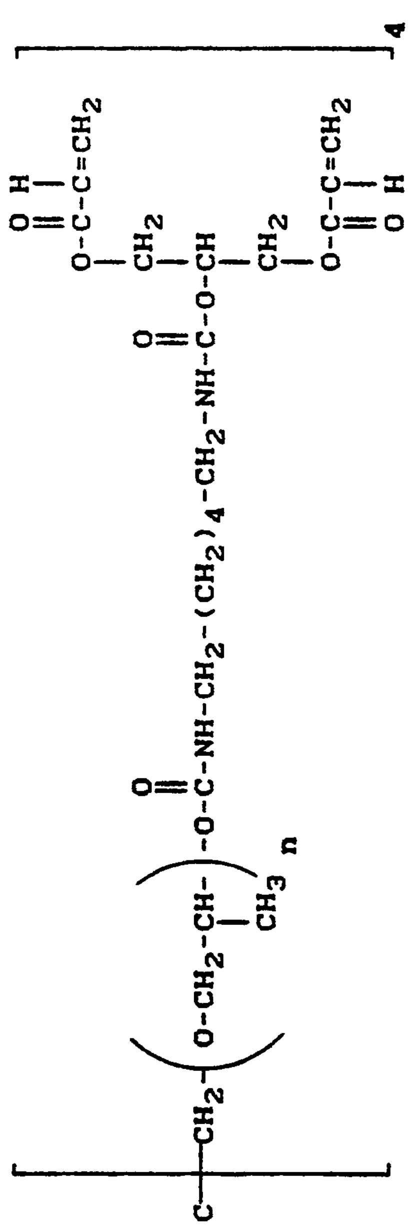

n = 1,225 (statistischer Mittelwert für 4 Ketten)

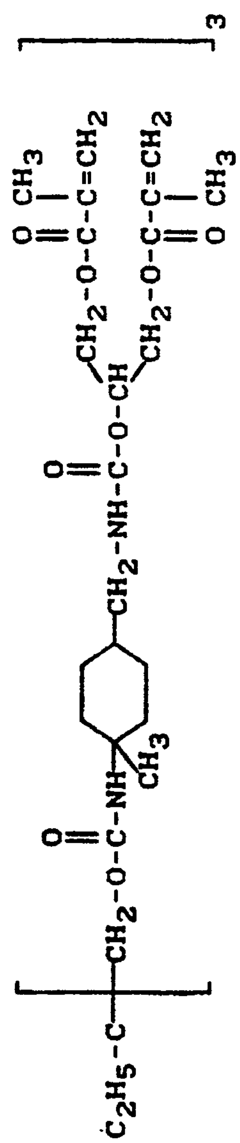

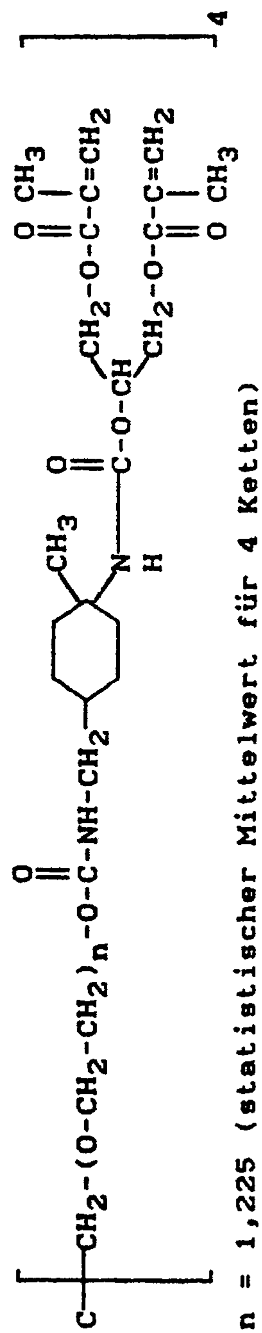

n = 1,225 (statistischer Mittelwert für 4 Ketten)

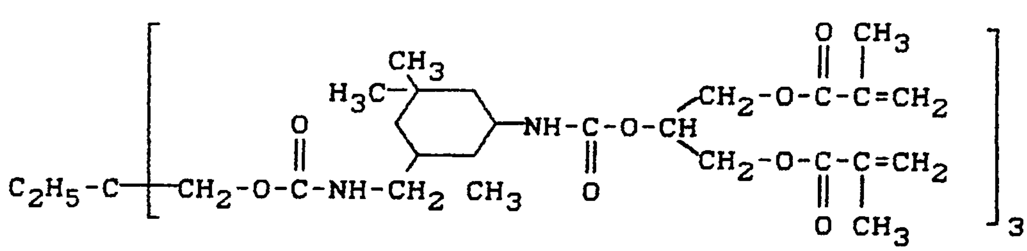

Besonders bevorzugt als Monomer wird das sogenannte Bis-GMA der Formel

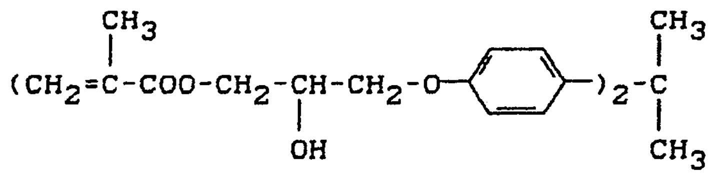 .

Selbstverständlich ist es möglich, Mischungen der verschiedenen (Meth)-Acrylsäureester, die Vernetzungen ausbilden können, einzusetzen. Beispielsweise seien Mischungen von 20 bis 70 Gew.-% Bis-GMA und 30 bis 80 Gew.-% Triethylenglykoldimethacrylat genannt.

Die erfindungsgemäßen Zubereitungen enthalten im allgemeinen 5 bis 80 Gew.-%, bevorzugt 10 bis 60 Gew.-% Carboxylverbindungen, bezogen auf den Carbonsäure-(meth)acryloylaminoalkylester.

Als weitere Komponente können die erfindungsgemäßen Zusammensetzungen Füllstoffe enthalten. Als Füllstoffe werden feine Pulver bevorzugt, die einen Teilchendurchmesser im Bereich von 0,1 bis 100 $\mu$m (gegebenenfalls auch in einer polydispersen Verteilung) haben. Füllstoffe können im Dentalbereich übliche Füllstoffe (R. S. Baratz, J. Biomat. Applications, Vol 1, 1987, S 316 ff) wie anorganische Gläser, Siliziumdioxid-, Aluminiumoxid- oder Quarzpulver sein.

Durch einen Anteil an Füllstoffen in den erfindungsgemäßen Zubereitungen entstehen Klebezemente, die besonders zur Befestigung von Brücken-, Kronen- und anderen Verblendmaterialen geeignet sind.

Der Anteil des Füllstoffs beträgt im allgemeinen 20 bis 80 Gew.-%, bevorzugt 40 bis 70 Gew.-%, bezogen auf die gesamte Zubereitung.

Die Adhäsivkomponenten gemäß dieser Erfindung können weiterhin bis zu 10 Gew.-% üblicher Zusätze wie Stabilisatoren, Inhibitoren, Lichtschutzmittel, Farbstoffe, Pigmente oder Fluoreszenzstoffe enthalten.

Die erfindungsgemäßen Zubereitungen können hergestellt werden, indem man den Carbonsäure-(meth)acryloylaminoalkylester und den Initiator und gegebenenfalls die anderen Komponenten durch kräftiges Rühren mischt.

Die Zubereitungen können auch lösungsmittelfrei vorliegen.

Die erfindungsgemäßen Zubereitungen können als Adhäsivkomponente zur Behandlung kollagenhaltiger Materialien verwendet werden.

In einer besonderen Ausführungsform konditioniert man das kollagenhaltige Material vor der Behandlung mit der erfindungsgemäßen Zubereitung mit einer Flüssigkeit mit einem pH-Wert im Bereich von 0,1 bis 3,5.

Diese enthalt im allgemeinen Säuren mit einem $pK_s$-Wert kleiner als 5 und gegebenenfalls eine amphotere Aminoverbindung mit einem $pK_s$-Wert im Bereich von 9,0 bis 10,6 und einem $pK_B$-Wert im Bereich von 11,5 bis 12,5. Folgende Säuren können z.B. in der Konditionierflüssigkeit enthalten sein: Phosphorsäure, Salpetersäure, Brenztraubensäure, Zitronensäure, Oxalsäure, Ethylendiamintetraessigsäure, Essigsäure, Weinsäure, Äpfelsäure, Maleinsäure.

Als amphotere Aminoverbindungen seien vorzugsweise Verbindungen der Formel

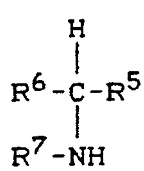

in der

$R^5$

für eine Carboxylgruppe steht,

$R^6$

Wasserstoff, gegebenenfalls durch Hydroxy, Thio, Methylthio, Carboxy, Amino, Phenyl, Hydroxy-phenyl oder die Gruppen

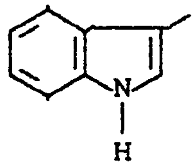 , 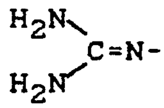 ,

substituierter Niederalkylrest,
$R^7$
Wasserstoff oder Phenyl bedeutet,
wobei die Reste $R^5$ und $R^7$ durch einen Propylrest verbunden sein können, oder
in der
$R^5$
für Wasserstoff steht,
$R^6$
für die Gruppe

$-B-NH_3X$,

in der

B für einen zweibindigen Alkylenrest mit 1 bis 6 Kohlenstoffatomen und
X für Halogen steht,

bedeutet und
$R^7$
Wasserstoff bedeutet,
genannt.

Beispielsweise seien die folgenden amphoteren Aminoverbindungen genannt: Glycin, Serin, Threonin, Cystein, Tyrosin, Asparagin, Glutamin, Alanin, Valin, Leucin, Isoleucin, Prolin, Methionin, Phenylalanin, Tryptophan, Lysin, Arginin, Histidin, N-Phenylglycin, Ethylendiaminhydrochlorid, Ethylendiaminhydrobromid, Propylendiaminhydrochlorid, Propylendiaminhydrobromid, Butylendiaminhydrochlorid, Butylendiaminhydrobromid, Leucinhydrochlorid und Histidinhydrochlorid.

Weiterhin kann die Konditionierflüssigkeit Stoffe aus der Gruppe der Polyethylenglykole und Metallhydroxide enthalten. Insbesondere können die oben aufgeführten mehrbasigen Säuren auch als teilweise Metallsalze eingesetzt werden solange freie Säurefunktionen verbleiben.

Konditionierflüssigkeiten, die mindestens eine der Säuren aus der Gruppe der Brenztraubensäure, Ethylendiamintetraessigsäure und Zitronensäure sowie gegebenenfalls eine amphotere Aminoverbindung aus der Gruppe des Glycins, N-Phenylglycins und Prolins enthalten, sind besonders bevorzugt.

Die Anwendung der erfindungsgemäßen Zubereitungen kann beispielsweise wie folgt durchgeführt werden:
Bei einer Zahnreparatur trägt man beispielsweise nach einer mechanischen Reinigung des kollagenhaltigen Zahnmaterials zuerst die Konditionierflüssigkeit mit etwas Wette auf, läßt eine kurze Zeit (beispielsweise 60 Sekunden) einwirken, spült das Zahnmaterial mit Wasser und trocknet es im Luftstrom. Dann trägt man die erfindungsgemäße Zubereitung beispielsweise mit einem kleinen Pinsel in einer dünnen Schicht auf und trocknet im Luftstrom. Nach der erfindungsgemäßen Behandlung trägt man die eigentliche Füllmasse, beispielsweise im Dentalbereich übliche Kunststoffüllungsmassen (K. Eichner, "Zahnärztliche Werkstoffe und ihre Verarbeitung", Bd. 2, S. 135 ff, Hüthig Verlag, 5. Aufl. 1985) auf.

In ähnlicher Weise können die erfindungsgemäßen Zubereitungen zur Befestigung von Kronen, Brücken und ähnlichen Hilfsmitteln verwendet werden.

Beispiel 1 bis 3 (Herstellung)

Die erfindungsgemäßen Klebemittel werden durch intensives Vermischen der in folgenden Beispielen aufgeführten Bestandteile erzeugt.

Beispiel 1
46 g Essigsäuremethacryloylaminomethylester der Formel

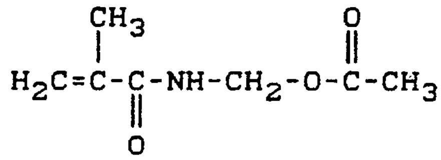

54 g Wasser
125 mg Campherchinon

Beispiel 2
40 g Essigsäuremethacryloylaminomethylester
42 g Wasser
18 g 25 gew.-%ige wäßrige Glutardialdehyd-Lösung
125 mg Campherchinon

Beispiel 3
35 g Propionsäuremethacryloylaminomethylester der Formel

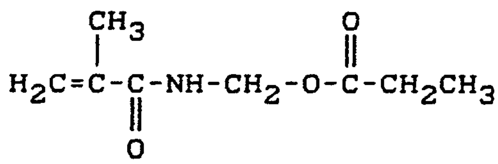

35 g Wasser
20 g 25 gew.-%ige wäßrige Glutardialdehyd-Lösung
10 g Tetrahydrofuran
125 mg Campherchinon

Beispiel 4 (Anwendung)

Die Eignung der Klebemittel entsprechend der Beispiele 1 bis 3 wird geprüft, indem die Zugbindungsfestigkeit des lichtaktivierten Kunststoffüllungsmaterials auf Basis mehrfunktioneller Methacrylsäureester und Bariumaluminiumsilikat LUMIFOR® Light Curing Composite Universal (U), auf Dentin bestimmt wird.

Für die Versuche werden extrahierte menschlische Zähne verwendet, die maximal 3 Monate nach der Extraktion in 1 % Chloraminlösung aufbewahrt wurden. Nachdem diese Zähne sorgfältig unter fließend Wasser gereinigt wurden, erfolgte die Aufbewahrung bis zur Einbettung in Expoxidharz (Lekutherm X 257) in physiologischer Kochsalzlösung.

Unter Verwendung von Schleifpapier unterschiedlicher Körnung wird der Zahn naß angeschliffen bis eine hinreichend große Dentinfläche zur Abbindung eines Kunststoffüllungsmaterialcylinders von Ø 3,5 mm freiliegt. Die freigelegte Dentinfläche wurde naß mit Siliziumcarbidpapier 600 abschließend präpariert.

Das Dentin wird nacheinander mit der EDTA-Konditionierflüssigkeit GLUMA® Cleanser (60 Sekunden Reinigung mit einem Wattepellet, Wasserspülung, Lufttrocknung) und dem Klebemittel (60 Sekunden Einwirkzeit, Lufttrocknung) vorbehandelt.

Zur Herstellung des Probekörpers für den Zugbindungsversuch, wird die wie beschrieben präparierte Dentinprobe in ein Stativ mit einer zylindrischen, teilbaren Teflonform gespannt. Diese insgesamt 5 mm hohe Teflonform ist in der oberen Hälfte konisch ausgeführt, so daß mit einem entsprechend geformten Adapter ein Zugversuch durchgeführt werden kann.

Ein Verschlußmaterial auf Basis mehrfunktioneller Methacrylsäureester BAYER RESIN L® wird in dünner Schicht mit einem Pinsel auf die vorbehandelte Dentinoberfläche aufgetragen und mit einem Luftstrom zusätzlich verteilt.

Das Verschlußmaterial wird zunächst im Abstand von 5 mm zur Dentinoberfläche mit einer Polymerisationsleuchte (Translux CL, Fa Kulzer) bestrahlt. Danach erfolgt ein incrementales Formfüllen und Lichtaktivieren des Kunststoffüllungsmaterials.

Die Lichtaktivierungszeit für das Kunststoffüllungsmaterial wird aufgrund des großen Volumens mit

insgesamt 160 Sekunden angesetzt.

Nach Abschluß der Lichtaktivierung wird der Probekörper entfernt und bis zum Zungversuch im Wasserbad bei 23° C gelagert.

Die Zugbindungsfestigkeit, Kraft beim Bruch der Probe dividiert durch das Kontaktareal zum Dentin, wurde mit einer Vorschubgeschwindigkeit von 1 mm/min. gemessen.

Die Bruchfläche am Dentin wird anschließend lichtmikroskopisch zur Beurteilung der Versagensursache überprüft. Hierbei waren vielfach Kohäsivfrakturen zu beobachten, d.h. die mit den erfindungsgemäßen Adhäsivkomponenten erzeugten Verklebungen waren fester als die verklebten Fügeteile selbst. Dies zeigt die gute Leistungsfähigkeit der erfindungsgemäßen Adhäsivkomponenten.

## Patentansprüche

1. Zubereitung enthaltend Carbonsäure-(meth)acryloylaminoalkylester (I) der Formel

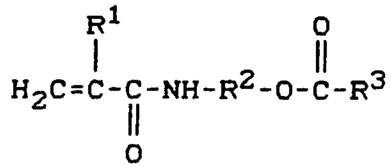

(I),

in der
$R^1$
Wasserstoff oder Methyl bedeutet,
$R^2$
Methylen oder Ethylen bedeutet
und
$R^3$
für Wasserstoff, Methyl oder Ethyl steht und gegebenenfalls Zusätze wie Initiatoren, Lösungsmittel und Füllstoffe als Adhäsivkomponente zur Behandlung kollagenhaltiger Materialien.

2. Zubereitung nach Anspruch 1, enthaltend Carbonsäure-(meth)acryloylaminoalkylester,
worin
$R^1$
Wasserstoff oder Methyl bedeutet,
$R^2$
Methylen bedeutet,
$R^3$
für Wasserstoff oder Methyl steht.

3. Zubereitung nach den Ansprüchen 1 und 2, dadurch gekennzeichnet, daß als Initiator ein Radikalbildner aus der Reihe der Mono- oder Dicarbonylverbindungen eingesetzt wird.

4. Zubereitung nach den Ansprüchen 1 bis 3, dadurch gekennzeichnet, daß der Carbonsäure-(meth)-acryloylaminoalkylester und der Initiator in einem Lösungsmittel gelöst sind.

5. Zubereitung nach den Ansprüchen 1 bis 4, dadurch gekennzeichnet, daß ein Coaktivator zugesetzt wird.

6. Zubereitung nach den Ansprüchen 1 bis 5, dadurch gekennzeichnet, daß als weitere Komponente eine Carbonylverbindung zugesetzt wird.

7. Zubereitung nach den Ansprüchen 1 bis 6, dadurch gekennzeichnet, daß als weitere Komponente (Meth)acrylsäureester, die Vernetzungen ausbilden können, zugesetzt werden.

8. Zubereitung nach den Ansprüchen 1 bis 7, dadurch gekennzeichnet, daß als weitere Komponente ein Füllstoff zugesetzt wird.

**9.** Verfahren zur Herstellung von Zubereitungen zur Verwendung als Adhäsivkomponente zur Behandlung kollagenhaltiger Materialien, dadurch gekennzeichnet, daß man Carbonsäure-(meth)-acryloylaminoalkylester der Formel

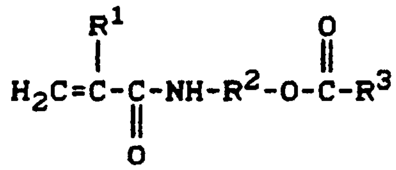

(I),

in der
$R^1$
Wasserstoff oder Methyl bedeutet,
$R^2$
Methylen oder Ethylen bedeutet
und
$R^3$
für Wasserstoff, Methyl oder Ethyl steht und gegebenenfalls Zusätze wie Initiatoren und Füllstoffe in einem Lösungsmittel mischt.

**10.** Verwendung Von Zubereitungen, enthaltend Carbonsäure-(meth)acryloylaminoalkylester der Formel

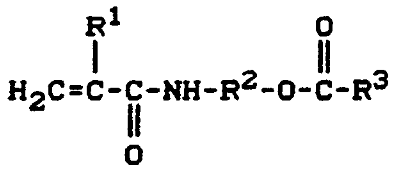

(I),

in der
$R^1$
Wasserstoff oder Methyl bedeutet,
$R^2$
Methylen oder Ethylen bedeutet
und
$R^3$
für Wasserstoff, Methyl oder Ethyl steht und gegebenenfalls Zusätze wie Initiatoren, Lösungsmittel und Füllstoffe als Adhäsivkomponente zur Behandlung kollagenhaltiger Materialien.

**11.** Verwendung von Zubereitungen nach Anspruch 10, nach einer Konditionierung des kollagenhaltigen Materials mit einer Flüssigkeit eines pH-Wertes von 0,1 bis 3,5.

**12.** Verwendung als Zubereitungen nach den Ansprüchen 10 und 11 als Klebemittel zur Befestigung von Zahnreparaturmaterialien am Zahn.

**13.** Verwendung der Zubereitung nach Anspruch 10 als Klebemittel in Knochenzement.